Europäisches Patentamt

European Patent Office

Office européen des brevets

(19)

(11) **EP 1 248 231 A1**

(12) **EUROPEAN PATENT APPLICATION**
published in accordance with Art. 158(3) EPC

(43) Date of publication:
09.10.2002 Bulletin 2002/41

(51) Int Cl.7: **G06N 3/12**, C12N 15/00

(21) Application number: 00985897.8

(86) International application number:
**PCT/JP00/09252**

(22) Date of filing: 26.12.2000

(87) International publication number:
**WO 01/048690 (05.07.2001 Gazette 2001/27)**

(84) Designated Contracting States:
**DE ES FR GB IT NL**

(30) Priority: 28.12.1999 JP 37293699

(71) Applicant: **JAPAN SCIENCE AND TECHNOLOGY CORPORATION**
**Kawaguchi-shi, Saitama 332-0012 (JP)**

(72) Inventors:
• **MOROHASHI, Mineo**
**Kita-ku, Tokyo 114-0024 (JP)**

• **KITANO, Hiroaki**
**Kawagoe-shi, Saitama 350-0036 (JP)**

(74) Representative: **Nicholls, Michael John**
**J.A. KEMP & CO.**
**14, South Square**
**Gray's Inn**
**London WC1R 5JJ (GB)**

(54) **NETWORK INFERRING METHOD**

(57) The present invention provides a method for predicting a topology stepwise by producing candidate networks that can reproduce a given data profile, evaluating these candidate networks, and predicting a network structure and parameters that can provide the data profile. According to the present invention, a regulatory network for a gene that may induce a certain phenomenon or a metabolic network indicating enzyme and protein reactions can be predicted based on gene expression or protein concentration data.

Fig. 5

EP 1 248 231 A1

**Description**

Technical Field

The present invention relates to a method for predicting a system that can be modeled as a network of separate correlating elements that interfere with one another, based on information that indicate the behavior or state of the network. The network predicting method of the present invention is useful in predicting, based on data regarding gene expression, protein concentration, or such associated with a biological phenomenon, the structure of a regulatory network that regulates a gene that induces this phenomenon, or a metabolic network that shows reactions of enzymes or proteins, and so on.

Background Art

Recent rapid progress in molecular biology is contributing to the accumulation of a large number of data on various organisms. Typical examples of such organisms include yeast, nematode, and *Drosophila*. For example, for nematode (C. elegans), the entire cell lineage has been predicted (Salston et al., Dev. Biol. 100, 64-119, 1983) , and the connections in the nerve system and the like have been identified (White et al., Phil. Trans. R. Soc. 314, 1-340, 1986). Further, in 1988, the entire genomic DNA nucleotide sequence was clarified (Science 282, 2012-2017, 1998). In addition to nematode, the entire genomic DNA nucleotide sequence has already been determined for model organisms such as yeast, *E. coli*, and mycoplasmas. It is expected that identification of the entire nucleotide sequence of human genomic DNA, which is the ultimate goal, will be completed within several years.

However, clarification of a nucleotide sequence is totally different from the characterization of genes and their functions and interactions. That is, the determination of the nucleotide sequence of a genome only identifies an aspect of the genome as a genetic material. In order to understand an organism based on genetic information, the effects of and the interaction between the individual genes contained in the determined nucleotide sequence must be clearly identified.

Identification of the functions of or interactions between genes is very difficult. For example, it is considered that typically a human cell is composed of 5, 000 to 7, 000 types of proteins. A proteome cannot be understood until these various types of proteins are individually identified, and the functions of and interaction therebetween are characterized. Most of the current molecular biological efforts focus on the identification of a gene associated with a target phenomenon and its transcript, and on the elucidation of an interaction that may induce this phenomenon. In order to understand the interaction between genes, however, an enormous number of combinations in which a large number of genes are connected together must be considered. It is very difficult to perform these operations manually. The present inventors aim to overcome this difficulty by introducing a computer, which has the ability to calculate.

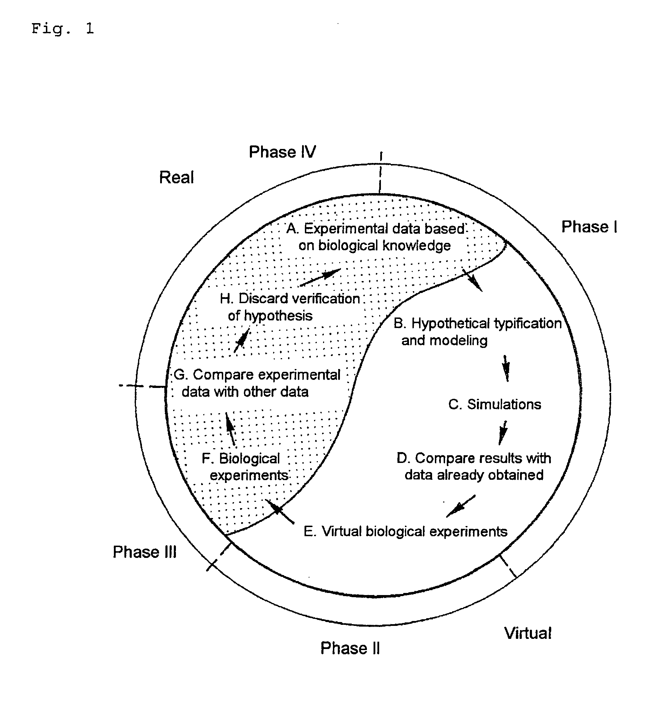

This concept is illustrated in Fig. 1. This drawing shows the relationship between a section that utilizes a computer (a virtual section) and a section that actually conducts biological experiments (a real section). In Fig. 1, when a phenomenon to be clarified is identified, a model is created on the computer (Fig. 1; phase IB) based on current biological knowledge (Fig. 1; phase IA). Alternatively, if a hypothesis has already been presented, it is fed to the computer. Such a model is used to carry out simulations (Fig. 1; phase IC), and the consistency between simulation results and observed data is evaluated (Fig. 1; phase ID). If the simulation results and the observed data do not agree with each other, there are two possibilities. One is that the simulations are imprecise. This problem can be solved by making the simulations more precise and thus more reliable. The other possible problem is the imperfection or incorrectness of the model. In this case, a model must be constructed that conforms to known experimental data.

Subsequently, this model is used to perform experiments on a computer if possible (Fig. 1; phase IIF). After phase II or without phase II if it is not possible, actual biological experiments are commenced to check whether or not the hypothetical model, or such, is correct (Fig. 1; phases III and IV).

In this manner, "biological reverse engineering" can be carried out. In addition to biology, a large number of fields require that a hypothetical model be verified by simulation on a computer.

For example, in molecular biology, a network is predicted based on data obtained using DNA chips, microarrays, PCRs, and so on, such as data on temporal variations in the amount of gene products or proteins. Alternatively, the data may be on the activities of a neural network obtained using electrophysiological experiments or voltage-sensitive pigments. If a computer can be used to predict models indicated by a network of interactions between genes, proteins, and enzymes, as well as a network of neurons, which are all behind the above data, it could be useful. However, the total number of models that can actually be derived from given data is enormous. Conventionally, only models that can be evaluated using the limited processing ability of human beings were considered. Alternatively, guesses were made relying on human intuition, which were then confirmed through biological experiments.

In contrast, the present inventors aim to limit the number of plausible models by using a computer to predict models (networks) that may generate data similar to given data. In Fig. 1, described above, this aim relates to phase I.

The term plausible model, as used herein, means a reasonable model that can be defined based on given data when a network is predicted. The reasonable model is useful in making a plan for the next experiment or constructing a new hypothesis.

The prediction of a model of interactions (network prediction) is required to identify a causative gene associated with a disease, to predict the effects of dosage, and so on. It can also be used to predict unknown genes or gene products. Further, verification of a hypothetical model based on computer simulations is an important analysis method not only in the biological fields, but also in fields such as traffic networks and economic systems..

In addition to biological phenomena, networks to be analyzed include artificial networks such as computer networks. For example, in the Internet, which can be called a "network of networks", simulations of traffic (the amount of information flowing through a network) are often performed. Prediction of throughput and the like are executed based on a large number of data, including the frequency of accesses to a router and such and the amount and size of flowing packets. These analyses correspond to the prediction of a network.

The present inventors established a network predicting method based on the above concept. That is, the present inventors proposed a method for generating a candidate network that can reproduce a target profile and screening the network using a mutation analysis (Morohashi et al., Proc. of European Conference on Artificial Life, 1999). The target profile, as used herein, refers to a set of data actually provided by a network to be clarified. Further, mutation analysis, as used herein, refers to a method for evaluating a candidate network based on data given by a network containing a mutant. With this method, data for the target profile or mutation analysis must be collected through actual experiments. That is, these data are "real". Consequently, experiments for mutation analysis must be repeated. This method involves a step of screening a single candidate. Accordingly, it was difficult to set a wide selection range.

Disclosure of the Invention

An objective of the present invention is to provide a network predicting method that can be implemented on a computer to allow efficient selection of hypothetically created models. More specifically, the objective is to provide a method for enabling a network to be efficiently predicted while reducing the number of actual experiments required.

Among the steps constituting the previously proposed network predicting method, the present inventors focused on mutation analysis. In order to effectively utilize computer-based simulations, it is important to minimize of the amount of data obtained through experiments . In other words, ideally, a maximum number of models should be screened based on a minimum number of experimental data to select a network having only a small error with the real world. Based on these concepts , the present inventors conducted extensive studies in order to realize a new network predicting method that requires no evaluation methods involving experiments such as mutation analyses.

In a library composed of candidate networks, the present inventors focused on networks that have only a small difference from a target profile. The present inventors then found that these networks frequently contain a common pathway. As used herein, the term "topology" means the physical structure constituting a network. The topology is composed of a set of pathways, which are lines joining together the elements constituting a network. Accordingly, in other words , a network is a topology composed of a set of pathways and involving various parameters. The present inventors further found that a network that can reproduce data can ultimately be constructed by extracting a common pathway, generating new candidate networks containing this pathway in their topologies, evaluating these networks, and repeating these steps, thus completing the present invention. That is, the present invention relates to the following network predicting method and apparatus therefor.

(1) a network predicting method that can reproduce data from given data produced by mutually related elements when the relationship between the elements can be described as a network, wherein the method comprises the steps of:

a) generating topologies that can reproduce the given data, and for each of the generated topologies, producing a parameter set that has the highest fitness to the given data, thereby obtaining candidate networks;
b) extracting a consensus pathway from the networks produced in step a) that meet a desired criterion;
c) generating topologies containing the consensus pathway extracted in step b) , and providing each of the generated topologies with a parameter set that has the highest fitness to the given data, thereby obtaining networks; and,
d) repeating steps b) and c) using the networks obtained in step c) as candidates until the scale of networks converges to a desired value, thereby obtaining a new network;

(2) the network predicting method according to (1) , wherein the desired criterion is evaluated based on fitness in step b);
(3) the network predicting method according to (1) , wherein the desired criterion is an ability to generate data that are similar to the data generated by a network containing the pathway in step b);

(4) the network predicting method according to (1), wherein the method comprises generating all topologies that can reproduce the given data in step a);

(5) the network predicting method according to (1), wherein the method comprises selecting networks that can reproduce the given data in step a), as networks that can reproduce the given data in step a);

(6) the network predicting method according to (5) , wherein the method comprises generating networks that reproduce data having only a small difference from the given data;

(7) the network predicting method according to (5) or (6) , wherein the step of obtaining networks in step a) and/ or c) comprises recombining parts of the structures of networks that reproduce data having only small differences from the given data;

(8) the network predicting method according to any one of (5) to (7), wherein obtaining networks in step a) and/or c) comprises searching structural neighborhoods of an arbitrary network to produce new networks;

(9) the network predicting method according to any one of (1) to (8), wherein obtaining networks in step a) and/or c) comprises predicting one parameter or a set of several different parameters for the same network;

(10) the network predicting method according to any one of (1) to (8), wherein obtaining candidate networks in step a) and/or c) comprises predicting parameters using at least one method selected from the group consisting of a genetic algorithm, simulated annealing, and a hill climbing method;

(11) the network predicting method according to any one of (1) to (10) , wherein the data generated by the elements is an expression profile of a gene;

(12) a network predicting apparatus that can reproduce data from given data produced by mutually related elements when the relationship between the elements can be described as a network, wherein the apparatus comprises the following means:

a) first storage means for storing networks each composed of a topology and a corresponding parameter set;
b) second storage means for storing candidate topologies or elements thereof and a corresponding parameter set;
c) means for generating topologies that can reproduce the given data, and for each of the generated topologies, producing a parameter set that has the highest fitness to the given data, thereby obtaining candidate networks, which are then stored in the first storage means;
d) means for extracting a consensus pathway from candidate networks which meet a desired criterion and are stored in the first storage means, and storing the consensus pathway in the second storage means;
e) means for generating topologies containing the consensus pathway stored in the second storage means by means d), providing each of the generated topologies with a parameter set that has the highest fitness to the given data, thereby obtaining networks, and storing the networks in the first storage means; and
f) means for providing the networks stored in the first storage means by the means e) as candidate networks for the means d) until a network to be predicted is obtained; and

(13) A storage medium storing a program that allows a computer system to implement a network predicting method according to any of (1) to (11).

The network according to the present invention is composed of three components: a topology, parameters, and fitness. These three components are collectively called a "triplet". The term "elements" as used herein encompasses substances constituting a network, and the topology indicates the relationship between these substances. Topology comprises a physical structure indicating the connections between the elements. This structure can be resolved into pathways. In other words, topology can be indicated as a collection of pathways. In particular, a pathway that is common to a plurality of topologies is called a "consensus pathway". The topology according to the present invention is modeled so that real data can be reproduced by the network. All the numerical values required for the modeling are called "parameters". The modeling is just an operation that applies a quantitative concept to a topology composed of only a physical structure. On the other hand, a set of data actually generated by a network is called a "target profile". Topologies with parameters enable differences from the target profile to be computed because they involve a quantitative concept. The magnitude of a difference from a target profile means the degree of similarity thereto, and in the present invention, this is referred to as "fitness".

The network predicting method provided in the present invention comprises the steps described below. A precondition of the present invention is that the relationship between mutually related elements can be described as a network. Accordingly, in other words, a target profile is generated as a result of interactions between the elements. The network predictingmethod of the present invention is characterized by comprising steps a) to d), described below, and aims to predict a network that can reproduce the target profile.

a) generating topologies that can reproduce the given data, and for each of the generated topologies, producing a parameter set that has the highest fitness to the given data, thereby obtaining candidate networks;

b) extracting a consensus pathway from the networks produced in step a) that meet a desired criterion;

c) generating topologies containing the consensus pathway extracted in step b), and providing each of the generated topologies with a parameter set that has the highest fitness to the given data, thereby obtaining networks; and,

d) repeating steps b) and c) using the networks obtained in step c) as candidates until the scale of networks converges to a desired value, thereby obtaining a new network.

In the present invention, the ability to represent the relationship between mutually related elements as a network means that at least some of the elements constituting the network are correlated with each other. Such a network can indicate, for example, interactions between genes constituting a certain cell. In this case, an expression profile of each gene contained in the cell corresponds to the target profile according to the present invention. The expression profile of a gene can be obtained by analyzing the mRNA or protein concentration as the expression level of the gene. More specifically, DNA chips can be used to obtain an mRNA expression profile. The use of DNA chips enables the expression levels of given nodes (i.e. probe sets fixed to the DNA chips) to be efficiently collected. A well-known alternative method is to obtain an expression profile of a protein using two-dimensional electrophoresis.

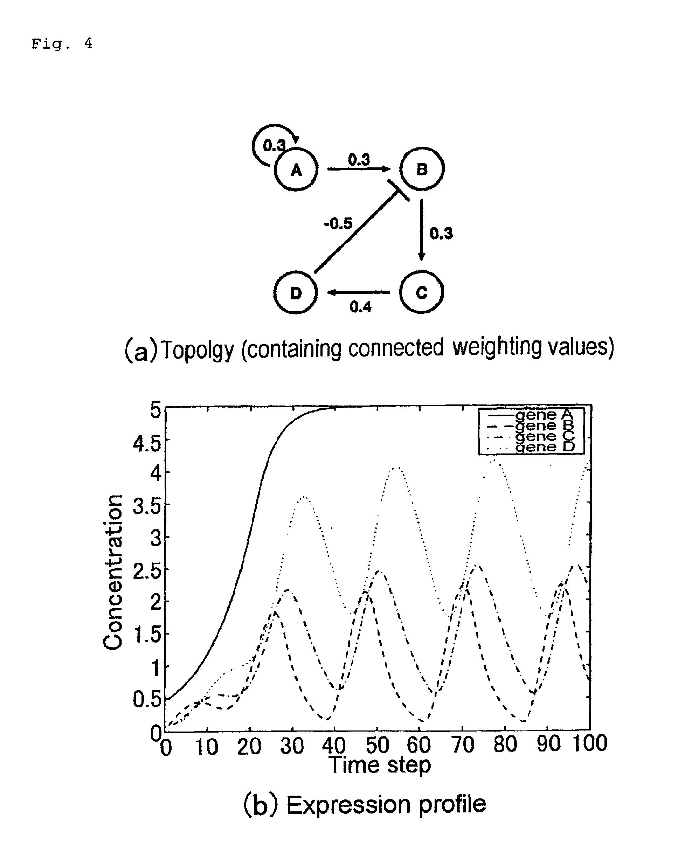

A network for reproducing a target profile can be represented by a triplet composed of the association between genes corresponding to the topology having genes as nodes, a parameter that indicates the level of interactions, and a fitness that indicates the degree of similarity to an expression profile. Fig. 4 shows a topology. Fig. 4a indicates the interaction between elements A, B, C, and D, i.e. indicates that element A activates element B, then element B activates element C, and finally element C activates element D, which, in turn inhibits element B. This figure also indicates that the element A activates itself. A network that can be predicted by the present invention and a triplet constituting the network are described below.

For example, in a network of interactions between enzymes, enzymatic actions using substrates as nodes constitute a topology, and the intensity of the activities and the level of the inhibitions are reduced to parameters. The fitness in this case represents the degree of similarity to a metabolic map of a cell or tissue. For data on the activities of a neural network obtained using electrophysiological experiments or voltage-sensitive pigments, and such, the synaptic connections between neurons correspond to a topology, and transmitted signals flowing through the synapses constitute parameters. The fitness in this case represents similarity to activities in the neural network. Furthermore, a traffic network and terminals connecting the elements of the network together, or an economic system and the elements constituting the system can also be described as a network according to the present invention.

It is normally unknown how many constituent elements of a network are associated with each other. Further, the association between elements may be completely unknown or partially identified.

In general, for the topologies generated in step a), diverse combinations are desirably generated in step a) in order to allow selection from as wide a range of topologies as possible. For example, generation of all assumable topologies is a preferred embodiment of the present invention. However, with a large number of elements to be considered, it is difficult to generate assumable topologies that can cover all the combinations between the elements. With an excessively large number of candidates for topologies, screening could be difficult. Accordingly, topologies can be selected as required. For example, topologies can be randomly selected from all assumable ones. The set of topologies generated in step a) are collectively referred to as a "topology pool".

Further, if the association between the elements constituting a network is already partially known, a reasonable screening can be achieved by generating topologies after this association fixed. For example, when analyzing a gene expression profile of a cell, the interaction between some genes is often partially known. In this case, it is rational to generate topologies generated with the already known part fixed. Conversely, it is expected that possible interactions that are entirely different from known relationships can be found by generating topologies while ignoring the commonly accepted theories about relationships between the elements. Moreover, even if the association between the elements constituting the network is unknown, topologies can be generated by experimentally fixing a part of the association based on a hypothesis. By applying the present invention to the thus generated various topologies, the plausibility of the hypothesis can be confirmed through comparison of the topologies.

In this case, with the network predicting method according to the present invention, a plurality of candidate networks can be generated allowing a wide range of assumable candidate networks to be obtained. This is a marked advantage over the well-known method for aiming to predict a single candidate network. Further, candidate networks predicted under various conditions can be more effectively compared with each other. For example, the ranking of a candidate network obtained through a prediction under certain conditions can be compared with that of a candidate network obtained through a prediction under other conditions.

The selection of topologies or networks in step a) may narrow the range of targets to be screened. However, the selection of candidates in step a) does not significantly affect the final results of the prediction of a network for the following reason. In the present invention, as described later, the step of predicting stepwise topologies that can reproduce given data is repeated. Accordingly, even if a particular candidate network is missing in a candidate group in a certain step, the resulting adverse effect is only partial. This is because the present invention repeats the step of extracting a consensus pathway, which is frequently found in the topologies. Consequently, if candidate networks are randomly selected, the consensus pathway can be extracted whether or not a particular topology is present.

The present invention further comprises generating topologies containing the consensus pathway and providing the generated topologies with parameters to obtain candidate networks again. The only condition imposed on the thus generated candidate networks is that they have the consensus pathway. The candidate networks may be generated for all randomly assumed topologies , or the present invention may further comprise frequently producing networks that reproduce data having only a small difference from the given data.

The step of frequently producing a desired network increases the probability of selecting candidate networks that can reproduce the given data. The present invention may further comprise partially recombining networks that reproduce data having only a small difference from the given data, to produce new networks. This can be implemented using a genetic algorithm (GA) and such.

The genetic algorithm was devised as a solution method. With the genetic algorithm, candidates that can achieve a certain object are produced by repeatingly evaluating a randomly generated candidate group from a particular viewpoint to select only candidates at a specific level or higher, and generating a new candidate group based on the selected candidates. Since this step is similar to the principle of the evolution of organisms, it is called the "genetic algorithm". Applying the genetic algorithm to the present invention, for example, enables one to produce artificial combinations between candidate networks having a high fitness. If this recombination results in a large number of networks with a high fitness, similar recombinations may be actively carried out based on the genetic algorithm.

The candidate networks thus obtained result from a wide and rough search of a network space. The term "network space", as used herein, means a space containing all assumable networks. A wide and rough search of a network space is called a "wide area search". In order to compensate for the wide area search, a search method for obtaining networks may comprise searching structural neighborhoods of an arbitrary topology to produce new topologies. Such a search method is called a "local search" as against the "wide area search". The local search can be carried out, for example, using simulated annealing (SA). Optimum networks can be efficiently selected by using the wide area search and local search in a hybrid form in order to generate candidate networks.

In the step of obtaining networks in step a) , one parameter or a set of plural different parameters can be predicted for the same topology. In order to predict the parameters, a well-known optimizing method ("Genetic Algorithm" supervised by Hiroaki KITANO and issued by Sangyo-Tosho, Ltd. (1993)) such as the genetic algorithm (GA), simulated annealing (SA), or the hill climbing method can be used. This enables quick prediction of optimum parameters for topologies that are required to reproduce the given data.

The hill climbing (HC) method carries out searches by selecting the most promising one of the possible search points. That is, this method finds the optimum solution in the neighborhoods of a search point. The simulated annealing (SA) method introduces a probabilistic transition into the concept of the HC method in order to avoid local minimums. The term "local minimum" means a solution that is optimum within a limited range but is not in a wide search range. The HC algorithm derives a solution by repeating local comparisons and thus tends to result in local minimums . The simulated annealing (SA) method differs from the HC algorithm in that when the neighborhoods of a search point $X_i$ are searched and a solution X is obtained, there is a possibility of $X_{i+1} = X$ even if an evaluated value for the solution X is worse than $X_i$. That is, if the new solution is better than the old one, the latter is replaced with the former. On the other hand, even if the new solution is worse than or equal to the old one, the latter is replaced with a different solution using a given probability. This operation leaves more solutions than the number of search points, and makes it more likely that not only local minimums but also a solution closer to the optimum one are obtained.

In step b) , a consensus pathway is extracted from the previously produced candidate networks that meet a desired criterion. The criterion to be met according to the present invention is that the difference from data generated by a network to be predicted is within an allowable range. In the present invention, the data generated by the network to be predicted is specifically called a "target profile" .

The differences between each network and the target profile can be compared using an arbitrary method. For example, comparison of fitness of the networks enables ranking using the magnitudes of differences between each of the candidate networks and the given data. The extracted consensus pathway is normally a structure frequently found in higher-ranking candidate networks, which have been ranked according to evaluation of fitness. The term "consensus pathway", as used herein, means a partial topology structure that can be extracted from a plurality of candidate networks. As described previously, in the present invention, one or more consensus pathways may be selected. For example, in a certain group of candidate networks, if a plurality of logically inconsistent structures are extracted from networks that meet the desired criterion, then the extracted structures are employed as candidates, and the subsequent process is divided into different processes for the respective candidates. In the present invention, when a consensus pathway is extracted and in the subsequent steps, extraction of the subsequent consensus pathway is repeated based on the first consensus pathway, the first extracted consensus pathway is expressed as being fixed.

The higher-ranking candidates preferably correspond to a group composed of 5% of all candidates that have a higher fitness calculated based on a total sum square method. The "high frequency" for such a group means when the consensus pathway is found in the candidates, for example, with a probability of 5% or more and more preferably 3% or more. A method for evaluating fitness based on a total sum square error in networks found by the total sum square method is described later in further detail. In other words, evaluation of fitness based on the total sum square error is equivalent to evaluation based on comparison in the time domain. In the present invention, comparisons can be made not only in the time domain but also using information in the frequency domain. The information in the frequency domain comprises an individual frequency components or a distribution thereof contained in the information in the time domain, and can be represented as a set of amplitudes and frequencies. These amplitudes and frequencies can be compared with those of a target using the TSS error or a correlation function.

In this case, the preset selection criterion of higher-ranking 5%, which is predetermined, can be varied depending on circumstances. For example, if elements to be extracted for a consensus pathway are dispersed among a large number of patterns and no significant difference in fitness is found between the higher-ranking 5% of all networks and the other networks, the higher-ranking 5% may be an insufficient selection range. In such a case, preferably the selection range for the consensus pathway may be widened to the extent in which a significant difference in fitness is found. Conversely, if the elements to be extracted for the consensus pathway concentrate in limited patterns, the selection range need not necessarily be widened.

In the present invention, one or more target profiles may be used, on which calculations of fitness are based. For example, data generated by a normal network (wild type) can be combined with a target profile comprising data provided by a network in which some elements have been artificially destroyed (mutant) . The use of several target profiles allows candidate networks to be evaluated in various aspects , thereby allowing easy selection from the candidates. Specifically, for example, by referencing a target profile based on a mutant, logically impossible candidates can be excluded from a group to be evaluated. Alternatively, more plausible candidates can be highly evaluated.

Whether or not the criterion for selecting a consensus pathway is met by networks can be judged not only by comparing fitness but also by evaluating whether or not networks containing a certain pathway can generate data which are similar to a target profile and which are also similar to each other. Data generated by a network containing the consensus pathway should be similar to the target profile. Therefore, by predicting whether or not a network containing a certain pathway can generate data similar to the target profile, it can be judged whether or not this pathway should be extracted as a consensus pathway. To achieve this, the consensus pathway can be fixed, for example, using the following algorithm. An important point of this method is the method of deriving a consensus pathway p. In this case, as a criterion for the derivation, a vector $\Gamma$ (gamma) of an N x N matrix (wherein number of network component is N) is defined. By using a topology having an elite number T corresponding to a high fitness from a topology pool, the elements $\gamma_{ij}$ of the vector $\Gamma$ are determined using Equation 1:

[Equation 1]

$$\gamma_{ij} = \sum_{k=1}^{T} w_{ij}^k \times A_i \times \beta_{ij}$$

wherein $w_{ij}^k$ denotes a connected weighting value for the i-th row and j-th column element in a topology k, $A_i$ denotes the average gene product concentration of a node i over all steps, and $\beta_{ij}$ denotes the sum of connection state values (1: connected, 0: unconnected) for the i-th row and j-th column elements in an elite.

In Equation 1, a result is obtained which reflects the connected weighting value, the expression product concentration of the node, and the inter-node connection frequency. However, this gives only very influential connections and fails to provide possible non-connections. Thus, a criterion $\gamma$ is introduced for non-connections. The elements $\gamma_{ij}$ are defined as follows:

[Equation 2]

$$\gamma'_{ij} = \frac{1}{\sum_{k=1}^{T} w_{ij}^{k} + \varepsilon} \times A_i \times (\mathrm{T} - \beta_{ij})$$

wherein E denotes a constant ($\fallingdotseq 0$).

Once the reference matrix $\Gamma$ has been derived, the element having the largest value in this matrix is assumed to be the most plausible pathway. That is, for example, if the element $\gamma$ (2,3) has the largest value in the matrix $\Gamma$ (activation) , it is assumed that node 2 provides strong activation control for node 3 . Hence , a search path as described later, or the like, can be used to recursively fix a consensus pathway by pre-determining the "number of pathways fixed during one cycle" based on the matrix $\Gamma$. "One cycle" according to the present invention includes steps a) to c), in which candidate networks are generated and evaluated to extract a consensus pathway, and a new network is generated based on this consensus pathway. As described later, the number of cycles constituting the network predicting method according to the present invention is not particularly limited.

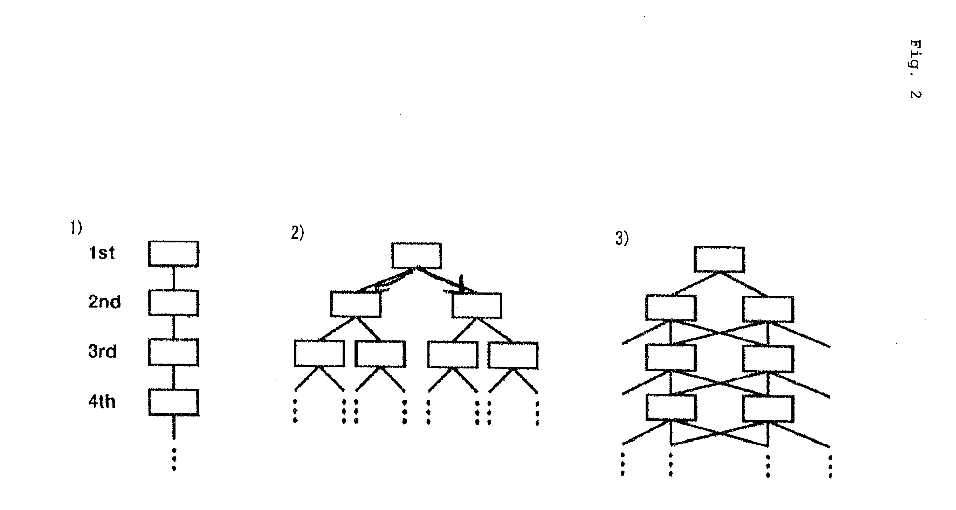

Various paths and hierarchical arrangements can be used to recursively search for a consensus pathway as shown in Fig. 2. In this drawing, the left search path is called a 1) "serial search path", the middle one, a 2) "pyramid search path", and the right one, a 3) "combinatorial search path". If there are several candidates for a consensus pathway, the pyramid or combinatorial search path can be used.

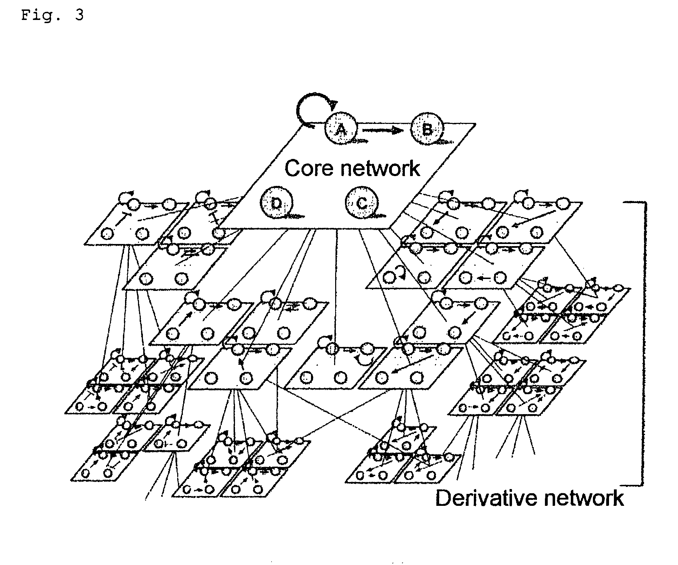

The serial search path is a search method for sequentially and serially adding consensus pathways. Further, with the pyramid search path, a plurality of consensus pathways are added in each hierarchy as candidates. The search method based on the pyramid search path is schematically shown in Fig. 3. This Fig. 3 shows a process used in the search method based on the pyramid search path to generate assumable networks (derivative networks) from a fixed network (core network). Furthermore, the combinatorial search path is a method for combining, in each hierarchy, consensus pathways from a higher hierarchy.

Subsequently in step c) , new topologies containing the consensus pathway are generated, and the generated topologies are each provided with a parameter set to obtain a group of secondary candidate networks. In order to generate a group of secondary candidate networks, the method for generating candidate networks in step a) can be directly applied. The group of secondary candidate networks are evaluated again as candidate networks in step b), and the step of extracting a consensus pathway is repeated. Each consensus pathway extracted in and after the second cycle must have a new structure compared to the preceding consensus pathway. That is, the extracted structure is different from that of the preceding consensus pathway, or contains the preceding consensus pathway and has more components than it. Regardless of the extracting method employed, consensus pathways indicated by combining together all pieces of information and including the latest one have an equal structure.

In the present invention, the network space is gradually narrowed as the steps of generating and evaluating candidate networks and extracting a consensus pathway are repeated. That is, the variations of candidate networks converge. The network predicting method according to the present invention can be completed when the scale of the network space is successfully reduced to an intended value. In other words, the network predicting method can be completed when the size of a group of networks that can reproduce given data is successfully reduced to a desired value.

The number of variations of candidate networks is minimized when the scale of a secondary candidate network space generated in step c) is the same as that obtained during the preceding cycle. At this time, the number of variations of networks, which comprise parameters and fitness provided for a consensus pathway used to generate these candidate networks, is minimum. Therefore, all the networks that can reproduce data can be selected by repeating the network predicting method of the present invention until the variations of candidate networks converge. However, the network predicting method according to the present invention can be ended at an arbitrary stage before the network space converges provided that its scale can be reduced to the desired value.

The present invention includes an apparatus that implements the above-described network predicting method and a storage medium storing a program that allows a computer system to implement the above-described network predicting method.

All of the cited prior art documents are incorporated herein by reference.

Brief Description of the Drawings

Fig. 1 is a diagram describing the use of a computer in molecular biology.
Fig. 2 is a diagram showing paths used to search for a consensus pathway; (1) shows a serial search path, (2) shows a pyramid search path, and (3) shows a combinatorial search path.
Fig. 3 is a diagram showing the concept of a pyramid search path through which a consensus pathway is searched.
Fig. 4 is a diagram showing a target profile generated by a network. In Fig. 4(a), the numbers indicate connected weighting values, and in Fig. 4 (b) , the horizontal axis indicates time, while the vertical axis indicates the concentration each gene product.
Fig. 5 is a diagram showing the concept of an iterative sampling method, which is an example of the network predicting method according to the present invention.
Fig. 6 is a chart showing the distribution of sum square TSS errors.
Fig. 7 is a diagram showing a hierarchical structure obtained experimentally.
Fig. 8 is a diagram showing an example of a gene and metabolic network.
Fig. 9 is a diagram showing an example of a target profile.

Best Mode for Carrying out the Invention

In order to demonstrate the effectiveness of the network predicting method according to the present invention, the following experiments were carried out. This predicting method is composed of repeating the processes of artificially generating a set of triplets (triplet pool) and extracting a candidate triplet therefrom. One process cycle comprising the generation and extraction is called a "sampling cycle". An "iterative sampling method" was applied to this extraction process; with this method, an essential topology that plays a key role in constructing a gene network is derived from a set of networks present in structural neighborhoods. This method is mainly characterized by focusing on the extraction of a more reliable topology from a set of candidate networks. The algorithm of the network predicting method based on the present invention is shown below.

1. Possible network topologies are generated, and for each of the topologies, parameters are optimized so as to agree with target data.
2. Each network is evaluated for fitness (using the TSS error and such).
3. A consensus pathway is derived from a T number of networks with a high fitness or from all weighted networks.
4 . The consensus pathway is fixed in accordance with a certain criterion.
5. Network topologies containing the already fixed consensus pathway are generated, and for each topology, the parameters are optimized.
6. The process returns to step 2.

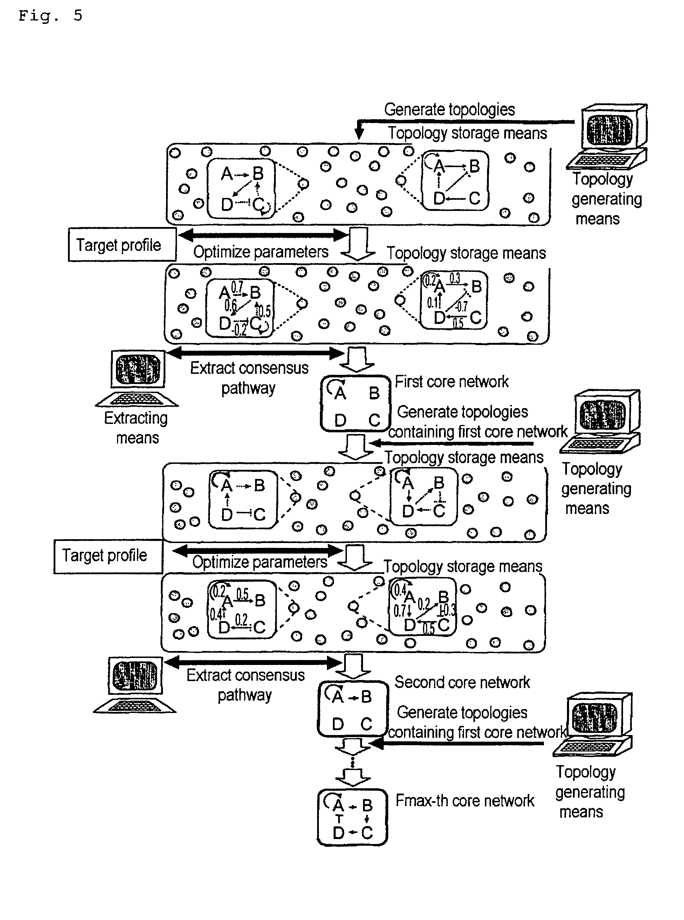

Fig. 5 shows this algorithm. Herein, the consensus pathway is a combined structure fixed as elements of a plausible network and is assumed to be a core network. During each cycle, a constant number of combined structures are fixed and added to the core network fixed during the preceding cycle, as an "Fth-order core network" if required. F denotes the number of iterations of the sampling cycle.
Further, this algorithm is indicated as follows using pseudocodes:

```
F=1;

G(F)={φ};

while (F≤Fmax){

Generate network topologies containing D=G(F);

Evaluate each network in D for fitness (using the TSS error or the
like);

Evaluate a consensus topology in D;

if (p is frequently found){

G(F+1)=G(F)U{ṗ};

}

F++;

}

Output G(F);
```

In this program, D denotes a set of candidate networks, G(F) denotes a core network at a hierarchical depth F, and p denotes a consensus pathway through which those of the candidates of a candidate group the number of which is defined by a function size S (p) are added to the core network. In this algorithm, if a first-order core network at F = 1 is to be generated, since G(F) = {φ}, a group of candidate networks are completely randomly generated and stored in the set D. Then, statistical evaluations are performed to extract a consensus pathway p as a first-order core network. Thus, one cycle is completed. Furthermore, for second-order networks at F = 2, derivative networks containing the first-order core network are stored as the candidate group in set D and then evaluated. The consensus pathway p is then added to the higher network, in this case, the first-order core network to obtain a second-order core network. Similar operations are repeated until the depth F reaches the value Fmax. That is , the highest hierarchy is not the only core network, and if networks below a certain hierarchy are to be searched, the networks in this hierarchy are assumed to be derivative, and functions are then added to these networks to generate a core network for the lower hierarchy.

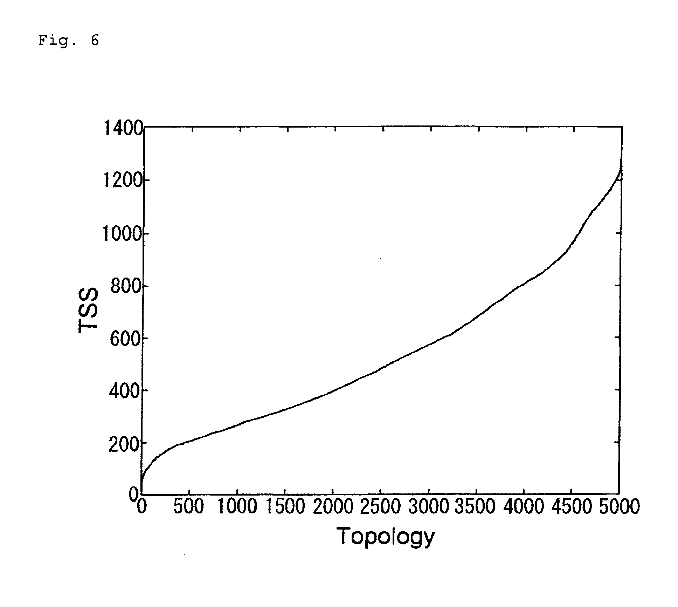

A triplet extracting process was experimentally executed by setting the size of G(F) at 5,000. The distribution of TSS errors is shown in Fig. 6. A consensus pathway P was extracted using an elite number E = 300, the cycle was repeated until F = 2, and the function size S(p) for each cycle was set at 2.

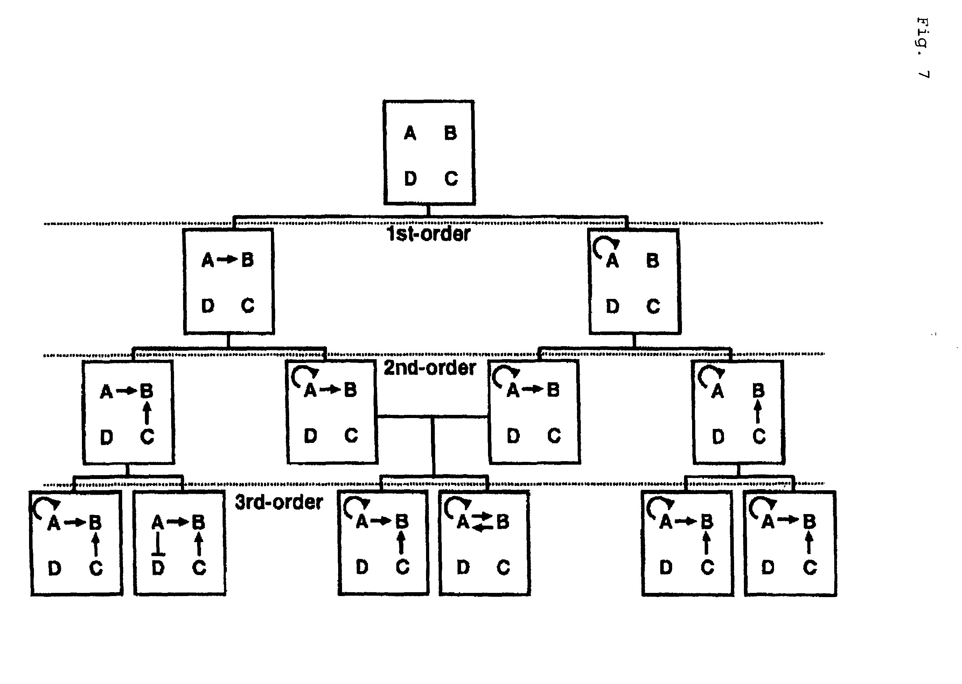

The $\Gamma$ (activation) (Equation 3) and $\Gamma$ (inhibition) (Equation 4) during the first cycle are shown below. Each of the underlined elements is the maximum value of the corresponding matrix. The hierarchical structure experimentally obtained is shown in Fig. 7.

[Equation 3]

$$\Gamma(activation) = \begin{pmatrix} 20606.2 & \underline{62648.4} & 9184.12 & 16817.8 \\ 10298.4 & 3726.79 & 5923.83 & 8764.97 \\ 14187.5 & 19064.7 & 4204.16 & 8081.3 \\ 3241.13 & 13953.1 & 1082.62 & 5183.11 \end{pmatrix}$$

[Equation 4]

$$\Gamma(inhibition) = \begin{pmatrix} 132.628 & 141.171 & 1082.3 & 4985.05 \\ 5448.63 & 554.033 & 1878.69 & 1177.37 \\ 862.98 & 1622.94 & 1193.72 & 2394.87 \\ 898.286 & \underline{11386} & 1519.07 & 0.279233 \end{pmatrix}$$

The extracted candidate networks included a candidate topology containing networks indicating the activation of A by itself and activation of B by A, which constitute a part of the target profile shown in Fig. 4(a). Thus, it is evident that the present invention enables prediction of a network configuration that efficiently generates a target profile.

The above-described process and a process of extracting candidate triplets will be specifically described taking a specific gene network as an example. This gene network generates an expression profile composed of time series data such as those shown in Fig. 4(b).

First, chromosomes were coded based on a connection matrix indicating the connection relations of a topology. The term chromosome as used herein is a term for the genetic algorithm. That is, the chromosome as used herein is obtained by coding a connected weighting as genes and a threshold. A network is constructed based on parameters coded on the chromosome. If this relationship is applied to the chromosome as a biological term, the parameters coded on the chromosome corresponds to genes , and the network generated based on the parameters corresponds to an expression type.

In this case, because Fig. 4 (a) shows four expression products, a topology of networks having four nodes will be considered. Such a topology is represented by a connection matrix C such as the one shown below.

[Equation 5]

$$C = \begin{pmatrix} 1 & 1 & 0 & 0 \\ 0 & 0 & 1 & 0 \\ 0 & 0 & 0 & 1 \\ 0 & -1 & 0 & 0 \end{pmatrix}$$

This topology has five connections, each of which must be weighted, so that chromosomes having four genotypes were generated. Furthermore, since the threshold of each node must be optimized, ultimately, chromosomes having 9 genotypes (five (number of connections) genotypes plus four (number of thresholds for anode) genotypes) will be generated. The allowable ranges (real values) of the weightings on the chromosomes and of the thresholds are shown in Table 1.

Table 1

| Connected weighting | |
|---|---|
| A→A | [0~1] |
| A→B | [0~1] |
| B→C | [0~1] |
| C→D | [0~1] |
| D→B | [-1~0] |

Table 1   (continued)

| Connected weighting | |
| --- | --- |
| Threshold for node | |
| A | [0~1] |
| B | [0~1] |
| C | [0~1] |
| D | [0~1] |

In this manner, a plurality of topologies that could achieve the expression profile shown in Fig. 4(b) were generated and stored in topology storage means (the first-order or top topology storage means in Fig. 5).

The genetic algorithm GA was used to optimize the parameters (weighting and threshold) for each of the stored topologies. First, N sets of parameters within the ranges determined for the topology were randomly generated to obtain an initial group of chromosomes. Table 2 shows an initial group for the topology determined by the above-described connection matrix. Each individual in the table is composed of a chromosome having five connected weightings and four thresholds as genes.

Table 2

| Connected weighting | Individual 1 | Individual 2 | Individual 3 | Individual N |
| --- | --- | --- | --- | --- |
| A→A | 0. 2 | 0. 3 | 0 ... 0. 3 | |
| A→B | 0. 4 | 0. 7 | 0. 2 ... 0. 2 | |
| B→C | 0. 2 | 0. 2 | 0. 5 ... 0. 6 | |
| C→D | 0. 2 | 0. 4 | 0. 7 ... 0. 2 | |
| D→B | -0. 1 | -0. 3 | -0. 6 ...-0. 5 | |
| Threshold value | | | | |
| A | 0. 6 | 0. 6 | 0. 3 ...0. 2 | |
| B | 0. 2 | 0. 2 | 0. 3 ...0. 4 | |
| C | 0. 3 | 0. 4 | 0. 3 ...0. 6 | |
| D | 0. 2 | 0. 5 | 0. 3 ...0. 2 | |

In this manner, for each topology, the chromosomes were determined in the form of lists. This initial group was optimized by applying the genetic algorithm GA thereto. The fitness of each parameter was used as an index for the optimization . The GA parameters used were a population size of 500, a generation of 300, a crossover rate of 0.8, and a mutation rate of 0.01, and an elite conservation strategy and a tournament selection strategy were used.

The fitness of the initial group was found by generating an expression profile for each individual based on the chromosome parameters and executing a process for the total sum square TSS error between the expression profile and the target profile. The total sum square TSS error process is described below.

As shown in Fig. 4 (b) , target profiles are provided as time series data with regard to concentration. Thus, the concentrations of the expression substances A, B, C, and D at a time t are represented as $L_{TA}(t)$, $L_{TB}(t)$, $L_{TC}(t)$, and $L_{TD}(t)$, respectively, and based on the parameters of the generated chromosomes, the concentrations of the expression substances calculated by Equation (1), described above, can be represented as $L_{EA}(t)$, $L_{EB}(t)$, $L_{EC}(t)$, and $L_{ED}(t)$. The total sum square TSS error between the concentration $L_{TA}(t)$, $L_{TB}(t)$, $L_{TC}(t)$, or $L_{TD}(t)$ and the concentration $L_{EA}(t)$, $L_{EB}(t)$, $L_{EC}(t)$, or $L_{ED}(t)$ can be expressed as shown below. The thus determined total sum square TSS errors were defined as the fitness of each generated network (chromosome).

[Equation 6]

$$TSS \quad = \quad \sum_{X}^{A,B,C} \sum_{t=0}^{T} \left( L_{TX}(t) - L_{EX}(t) \right)^2$$

The thus determined fitness can be utilized to optimize the parameters (connected weighting and threshold) for each topology using the genetic algorithm GA. That is, a triplet pool composed of a collection of optimized triplets can be constructed by ranking the topologies based on the fitness, and selecting and storing those having a higher fitness, in the topology storage means (the first-order topology storage means, used after the parameter optimization).

Then, candidate networks can be predicted by using the constructed triplet pool to compare $\Gamma$ based on the equations described previously. More specifically, higher elements each having a larger $\Gamma$ (a, activation) or $\Gamma$ (i, inhibition) value are extracted as many as the number of consensus pathways fixed during one cycle. For example, if one consensus pathway is fixed during one cycle, elements each having the largest $\Gamma$ (a) or $\Gamma$ (i) value are selected as a consensus pathway. From the second cycle, the number of consensus pathways fixed is sequentially increased, and this operation is repeated for a required number of cycles.

(Other Applications)

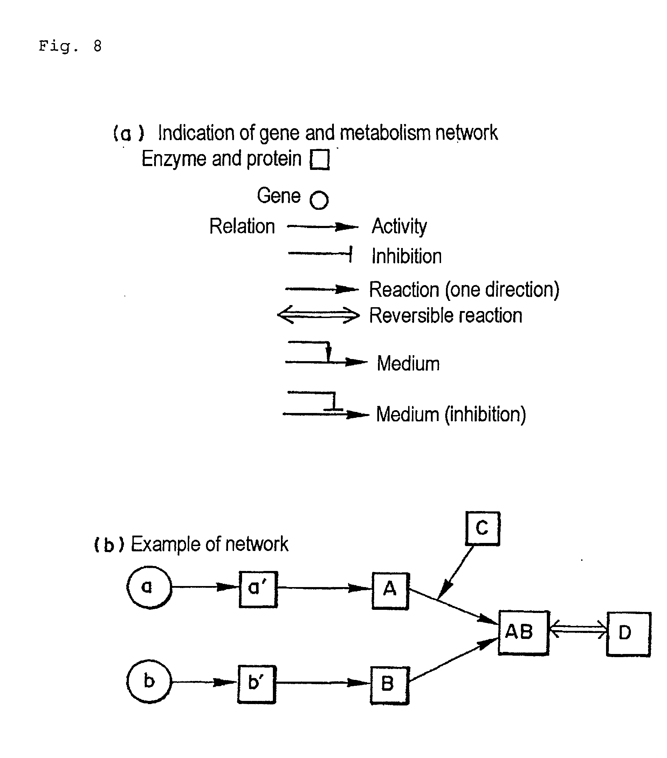

In the above description and examples, a gene network was used as an example. The present invention is also applicable to, for example, a gene and metabolic network. Fig. 8 shows such a gene and metabolic network. Genes as well as enzymes and proteins in metabolism are denoted by nodes, with relations such as activation, inhibition, and mediation denoted by links. A network using these nodes and links are shown in Fig. 8(a). The above-described process is applied to the topology shown in Fig. 8(b).

In the above description, a gene network and a metabolic network are shown, but the present invention is also applicable to a neural network and such. The data (target profile) required in this case is neuron action potential and such.

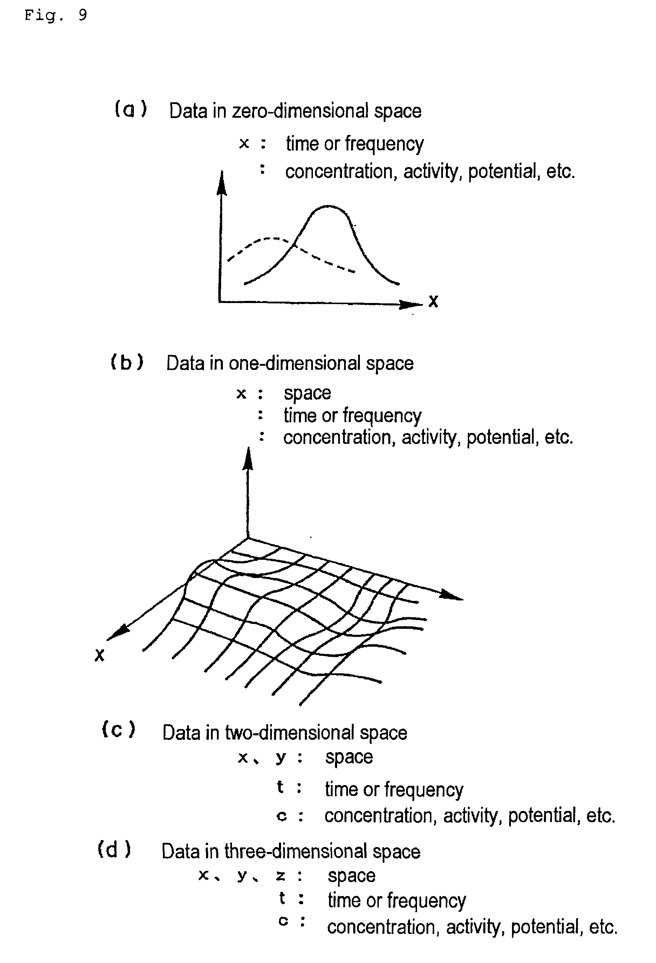

Thus, the method of the present invention can be used in various manners. Fig. 9 shows various examples of applicable target profiles. Fig. 9 (a) shows when the target profile consists of zero-dimensional space data . For example, these data vary with time or frequency. The data may be, for example, concentration, activity, or potential. Fig. 9(b) shows one-dimensional space data. The data in this case may be a quantity dependent on the space x and time or frequency. Such a quantity may be concentration, activity, or potential. The target profiles shown in Figs 9(c) and 9(d) are two- and three-dimensional space data. These data may be quantities dependent on a two- (x, y) and three- (x, y, x) dimensional spaces as well as time or frequency. Such quantities may be concentration, activity, potential, and such. In this manner, the target profile can be provided in various data forms.

As described above, the present invention is effective when a target profile is present and the network configurations generated by the target profile are predicted.

The present invention may be applied not only to a standalone computer system but also to, for example, a client server system composed of a plurality of computers.

The configuration of the present invention can be achieved by allowing the system to read out a program relating to the present invention, from a storage medium in which the program is stored and then executing the program. This storage medium may be a DVD, an MD, an MO, a floppy disk, a CD-ROM, a magnetic tape, a ROM cassette, or such.

Industrial Applicability

As described above, the present invention is very effective when a target profile is present as data and the network configurations generated by the target profile are predicted. In particular, the present invention can be effectively applied to predict a molecular biological model such as a gene network or a metabolic network.

The present invention enables a network to be predicted based on at least one data profile. Accordingly, the number of experimental steps required to obtain a data profile can be minimized. Alternatively, combining mutant-based target profiles together enables candidate networks to be more easily selected.

Further, in the present invention, a plurality of candidate networks can be easily evaluated if required. Therefore, a wide selection range is always provided, thereby allowing more diverse possibilities to be efficiently analyzed.

The present invention is particularly useful for a method for predicting a network using as a target profile a gene expression profile obtained using DNA chips. Each of the DNA chips has probes (a probe set) for a group of genes densely fixed thereto. DNA chips that are fixed with the same probe set produce highly reproducible analysis results provided that the other conditions for the chips are the same. That is, networks according to the present invention provide an expression profile based on networks having a common node. For analysis of an expression profile using DNA chips, a standard is set so that the analysis results of different experiments can be compared with each other. For example, the results of different experiments can be easily compared with each other by adapting hybridization or washing conditions, which markedly affect the results, to the predetermined common standard in advance.

Using the thus collected DNA-chip-based analysis results, a network is first predicted based on the analysis results for a first cell. Furthermore, a network is predicted based on an expression profile obtained from a second cell. If the DNA chips used comprise the same probe set, the network predicting method according to the present invention can be achieved with both expression profiles integrated together. Thus, candidate networks can be more efficiently selected by storing a plurality of expression profiles obtained using certain DNA chips and carrying out the network predicting method of the present invention based on the stored expression profiles. The main advantage of such an analysis method is that by storing experimental results from various research facilities in one location, analysis results obtained using DNA chips can be easily integrated.

Furthermore, by storing various expression profiles composed of the same nodes, the expression profiles of various genes can be analyzed using a computer. As a result, the computer can be used to predict an expression profile that may be obtained when a certain gene is inhibited, and an experiment plan can be made for experiments using the same DNA chips to experimentally confirm the prediction. As described above, the network predicting method of the present invention is particularly useful in analyzing expression profiles of genes using DNA chips.

**Claims**

1. A network predicting method that can reproduce data from given data produced by mutually related elements when the relationship between the elements can be described as a network, wherein the method comprises the steps of:

   a) generating topologies that can reproduce the given data, and for each of the generated topologies, producing a parameter set that has the highest fitness to the given data, thereby obtaining candidate networks;
   b) extracting a consensus pathway from the networks produced in step a) that meet a desired criterion;
   c) generating topologies containing the consensus pathway extracted in step b) , and providing each of the generated topologies with a parameter set that has the highest fitness to the given data, thereby obtaining networks; and,
   d) repeating steps b) and c) using the networks obtained in step c) as candidates until the scale of networks converges to a desired value, thereby obtaining a new network.

2. The network predicting method according to Claim 1, wherein the desired criterion is evaluated based on fitness in step b).

3. The network predicting method according to Claim 1, wherein the desired criterion is an ability to generate data that are similar to the data generated by a network containing the pathway in step b).

4. The network predicting method according to Claim 1, wherein the method comprises generating all topologies that can reproduce the given data in step a).

5. The network predicting method according to Claim 1, wherein the method comprises selecting networks that can reproduce the given data in step a), as networks that can reproduce the given data in step a).

6. The network predicting method according to Claim 5, wherein the method comprises generating networks that reproduce data having only a small difference from the given data.

7. The network predicting method according to Claim 5 or 6, wherein the step of obtaining networks in step a) and/ or c) comprises recombining parts of the structures of networks that reproduce data having only small differences from the given data.

8. The network predicting method according to any one of Claims 5 to 7, wherein obtaining networks in step a) and/ or c) comprises searching structural neighborhoods of an arbitrary network to produce new networks.

9. The network predicting method according to any one of Claims 1 to 8, wherein obtaining networks in step a) and/or c) comprises predicting one parameter or a set of several different parameters for the same network.

10. The network predicting method according to any one of Claims 1 to 8, wherein obtaining candidate networks in step a) and/or c) comprises predicting parameters using at least one method selected from the group consisting of a genetic algorithm, simulated annealing, and a hill climbing method.

11. The network predicting method according to any one of Claims 1 to 10, wherein the data generated by the elements is an expression profile of a gene.

12. A network'predicting apparatus that can reproduce data from given data produced by mutually related elements when the relationship between the elements can be described as anetwork, wherein the apparatus comprises the following means:

a) first storage means for storing networks each composed of a topology and a corresponding parameter set;
b) second storage means for storing candidate topologies or elements thereof and a corresponding parameter set;
c) means for generating topologies that can reproduce the given data, and for each of the generated topologies, producing a parameter set that has the highest fitness to the given data, thereby obtaining candidate networks, which are then stored in the first storage means;
d) means for extracting a consensus pathway from candidate networks which meet a desired criterion and are stored in the first storage means, and storing the consensus pathway in the second storage means;
e) means for generating topologies containing the consensus pathway stored in the second storage means by means d), providing each of the generated topologies with a parameter set that has the highest fitness to the given data, thereby obtaining networks, and storing the networks in the first storage means; and
f) means for providing the networks stored in the first storage means by the means e) as candidate networks for the means d) until a network to be predicted is obtained.

13. A storage medium storing a program that allows a computer system to implement a network predicting method according to any of Claims 1 to 11.

Fig. 1

Phase IV

Real

Phase I

A. Experimental data based on biological knowledge

H. Discard verification of hypothesis

B. Hypothetical typification and modeling

C. Simulations

G. Compare experimental data with other data

D. Compare results with data already obtained

F. Biological experiments

E. Virtual biological experiments

Phase III

Virtual

Phase II

Fig. 2

Fig. 3

Core network

Derivative network

Fig. 4

(a) Topolgy (containing connected weighting values)

(b) Expression profile

Fig. 5

Fig. 6

Fig. 7

EP 1 248 231 A1

Fig. 8

(a) Indication of gene and metabolism network
    Enzyme and protein ☐

Gene ○

Relation ——▶ Activity

—— Inhibition

——▶ Reaction (one direction)

⟺ Reversible reaction

——▶ Medium

——▶ Medium (inhibition)

(b) Example of network

Fig. 9

(a) Data in zero-dimensional space

x : time or frequency
: concentration, activity, potential, etc.

(b) Data in one-dimensional space

x : space
: time or frequency
: concentration, activity, potential, etc.

(c) Data in two-dimensional space

x、y : space
t : time or frequency
c : concentration, activity, potential, etc.

(d) Data in three-dimensional space

x、y、z : space
t : time or frequency
c : concentration, activity, potential, etc.

EP 1 248 231 A1

## INTERNATIONAL SEARCH REPORT

| | International application No. |
|---|---|
| | PCT/JP00/09252 |

**A. CLASSIFICATION OF SUBJECT MATTER**
Int.Cl$^7$   G06N3/12, C12N15/00

According to International Patent Classification (IPC) or to both national classification and IPC

**B. FIELDS SEARCHED**

Minimum documentation searched (classification system followed by classification symbols)
Int.Cl$^7$   G06N3/12, C12N15/00

Documentation searched other than minimum documentation to the extent that such documents are included in the fields searched
Jitsuyo Shinan Koho          1922-1996     Toroku Jitsuyo Shinan Koho  1994-2001
Kokai Jitsuyo Shinan Koho   1971-2001     Jitsuyo Shinan Toroku Koho  1996-2001

Electronic data base consulted during the international search (name of data base and, where practicable, search terms used)
JICST DATABESE (JOIS)

**C. DOCUMENTS CONSIDERED TO BE RELEVANT**

| Category* | Citation of document, with indication, where appropriate, of the relevant passages | Relevant to claim No. |
|---|---|---|
| Y | WO, 90/11568, A1 (HONEYWELL INC.),<br>04 October, 1990 (04.10.90),<br>Claims<br>& US, 5140530, A     & EP, 465489, B1<br>& JP, 2881711, B2 | 1-13 |
| Y | Tetsushi YATA et al., "Kakure MarKov Model to Identeki Algorithm ni yoru DNA Hairetsu no Signal Pattern Chushitsu", Transactions of Information Processing Society of Japan(IPSJ), Vol. 37, No. 6, 15 June, 1996 (15.06.96), pp.1117-1129 | 1-13 |
| Y | Hiroaki KITANO ed., "Identeki Algorithm 2", (Japan), Sangyo Tosho K.K., (08.05.95), pp. 18-33 | 1-13 |
| A | John Yen, et al., "A Hybrid Approach to Modeling Metabolic Systems Using a Genetic Algorithm and Simplex Method",IEEE TRANSACTIONS ON SYSTEMS,MAN, AND CYBERNETICS PART B: CYBERNETICS, Vol.28, No.2, April 1998 (30.04.98), pp.173-191 | 1-13 |

☐ Further documents are listed in the continuation of Box C.       ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28 March, 2001 (28.03.01) | 10 April, 2001 (10.04.01) |

| Name and mailing address of the ISA/ | Authorized officer |
|---|---|
| Japanese Patent Office | |
| Facsimile No. | Telephone No. |

Form PCT/ISA/210 (second sheet) (July 1992)

25